# DEMANDE DE BREVET EUROPEEN

(11) **EP 1 829 558 A1**
(43) Date de publication de la demande: **05.09.2007**
(21) Numéro de dépôt: 07300815.3
(22) Date de dépôt: 22.02.2007
(51) Int. Cl.: A61L 2/18, A01N 37/16, A61M 1/16, C11D 3/39

(54) **Nouvelle composition nettoyante, decalcifiante et desinfectante pour les generateurs de dialyse**

(30) Priorité: 03.03.2006 FR 0601917
(71) Demandeur: S.E.P.P.I.C., SOCIETE D'EXPLOITATION DE PRODUITS POUR LES INDUSTRIES CHIMIQUES, 75007 Paris (FR)
(72) Inventeur: Pierre, Marie-Pierre, 71640, Saint-Jean-de-Vaux (FR); Mabille, Jean-Benoit, 71100, La Charmee (FR); Le Rouzic, Daniel, 95120, Ermont (FR); Gamet, Jean-Claude, 71460, Saint-Martin-du-Tartre (FR)
(74) Mandataire: Conan, Philippe Claude

(57) **Abrégé**

Composition comprenant pour 100 % de sa masse, essentiellement :
de 0,50% massique à 1,50 % massique d'acide peracétique,
de 3,00% massique à 15,00% massique de peroxyde d'hydrogène,
de 3,00% massique à 15,00% massique d'acide acétique
de 0,10% massique à 1,00% massique d'acide nitrique concentré
de 0,001 % massique à 0,20% massique d'agent tensioactif non ionique
de 0,0 1 % massique à 0,10% massique d'oxyde d'amine,
de 0,01 % massique à 0,20% massique d'agent stabilisant, et

de l'eau pour compléter à 100% massique ; son utilisation pour nettoyer, décalcifier et désinfecter des équipements de dialyse entre deux séances successives de dialyse, sans traitement complémentaire autre qu'un rinçage dudit équipement de dialyse avec de l'eau osmosée ; les procédés pour sa mise en oeuvre.

## Description

L'invention concerne l'entretien des appareils de dialyse et plus particulièrement leur traitement entre deux utilisations.

Le principe général de la dialyse extra-corporelle repose sur des échanges à travers une membrane semi-perméable, entre le sang à épurer et un dialysât de composition électrolytique proche du liquide extracellulaire normal, qui est donc constitué d'eau et d'électrolytes en concentrations suffisantes pour que la concentration en électrolytes dans le sang en fin de dialyse, soit normale. Pour que des échanges efficaces puissent se faire, il faut en permanence renouveler le sang et le dialysât, car la vitesse de diffusion ou de dialyse est fonction de la différence de concentrations entre ces deux liquides.

Tout appareil de dialyse est constitué d'un générateur, d'un dialyseur, appelé aussi rein artificiel et d'un circuit d'eau. Cet ensemble d'éléments sera appelé dans l'exposé suivant, chaîne d'hémodialyse.

Le générateur a pour fonctions, d'assurer la préparation d'un dialysât réchauffé à 37°C, en mélangeant l'eau, une solution d'électrolytes dont la concentration ne permet pas la prolifération d'agents infectieux et une solution tampon et de faire circuler le sang et le dialysât tout en contrôlant les paramètres de dialyse. L'eau utilisée dans ces appareils devant être très pure et conforme à la Pharmacopée européenne, elle subit un traitement qui comporte une succession d'étapes, comme la filtration, le passage sur résines échangeuses d'ions, le passage sur charbon actif et pour terminer l'osmose inverse. Il existe différents types de générateurs ; il y a. ceux utilisant la technique du dialysât en circuit ouvert et qui permettent d'obtenir un dialysât de meilleure qualité bactériologique que ceux utilisant la technique dite en circuit fermé dans laquelle il y a re-circulation du dialysât

Le dialyseur a pour fonction d'éliminer l'eau et les déchets organiques provenant du sang du corps humain et qui migrent du sang vers le dialysât à travers la membrane semi-perméable. Cette membrane semi-perméable peut être disposée en plaques, en compartiments empilés les uns sur les autres, en forme de bobines, en compartiment unique enroulé en spirale autour d'un axe ou en ensembles de capillaires ; cette membrane peut être organique en cellophane, cuprophane, polysulfone, polypropylène, polyamide, polyacétate ou en copolymère d'acrylonitrile. Le dialyseur est livré stérile et est souvent à usage unique comme en France ; cependant dans certains pays le dialyseur est réutilisé.

Comme types de dialyseurs, on peut citer les reins bobines, comprenant un enroulement parallèle de deux feuillets de membranes dialysantes en spirale autour d'un axe, le sang circulant à l'intérieur de l'enveloppe formée par les deux feuillets de la membrane et le dialysât à l'extérieur, les reins à fibres creuses opposant une résistance très faible au passage du sang ou les reins à plaques comprenant 15 à 20 feuillets assemblés en plaques.

L'utilisation de solutions aqueuses d'acide peracétique pour désinfecter tous les éléments de la chaîne de dialyse est décrite depuis de nombreuses années. On peut citer par exemple L.J. Fischbach, AAMI Technol. Anal Rev. (1985), pages 15 à 18 ; Von SpröBig et al. Dtsch. Ges.wesen 27 (1972), H 23 pages 1085-1089 ; ou les demandes de brevet européen publiées sous les numéros EP 0 370 850, EP 0 873 687 et EP 1 068 873.

Cependant, le souhait de la clientèle tant hospitalière, associative ou privée de procéder à des séances de dialyse à la fois plus courtes et plus efficaces, a conduit les fabricants d'équipements de dialyse à mettre en oeuvre d'autres techniques d'hémodialyse que l'hémodialyse conventionnelle. Il y a notamment l'hémofiltration et l'hémodiafiltration.

L'hémodialyse conventionnelle : selon cette méthode, le transfert des solutés s'opère principalement par diffusion, tandis que celui du sodium et de l'eau s'opère sur un mode principalement convectif.

L'hémofiltration : selon cette méthode, le transfert des solutés est purement convectif. L'hémofiltration impose le recours à une membrane de haute perméabilité. La balance volémique (ou volume total du sang) du patient est maintenue, en réinjectant dans le circuit sanguin une solution de substitution de composition voisine de celle d'un ultratfiltrat plasmatique normal à un débit équivalent à celui du débit d'ultrafiltration, diminué du débit correspondant à la perte de poids désirée.

L'hémodiafiltration : l'hémodiafiltration combine à la fois les propriétés de l'hémodialyse conventionnelle et de l'hémofiltration ; selon cette méthode, le transport des solutés est d'une part par diffusion, ce qui assure une soustraction efficace des substances de déchets de faible poids moléculaire, et d'autre part convectif, ce qui accroît l'extraction des solutés de poids moléculaire élevé. L'hémodiafiltration nécessite donc à la fois un dialysât et une solution de substitution.

Les techniques d'hémofiltration et d'hémodiafiltration en ligne nécessitent des volumes importants de solution de substitution. Elles reposent sur la production extemporanée de cette solution à partir du dialysât affluent. Mais ces nouvelles techniques, qui utilisent des membranes à haute perméabilité, induisent l'apparition de dépôts protéiques et lipidiques issus du sang des patients, dans les circuits dialysât des générateurs. Ces dépôts s'accumulent jusqu'à obstruer les réseaux d'évacuation d'eau et bloquer le fonctionnement de l'équipement de dialyse.

A ces évolutions technologiques, se sont ajoutées les évolutions d'ordre réglementaire plus contraignantes relatives à l'évaluation de l'efficacité des désinfectants classés : "Dispositifs Médicaux".

Ces évolutions imposent donc la recherche et le développement de nouveaux procédés ou produits qui conduisent à une hygiène totale des équipements de dialyse, c'est-à-dire un nettoyage, une décalcification et une désinfection irréprochables de tous les éléments de la chaîne de dialyse.

Si les solutions d'acide peracétique sont largement utilisées pour leur de bonnes propriétés désinfectantes, elles ne sont par contre pas suffisamment décalcifiantes et absolument pas détergentes.

C'est la raison pour laquelle, les équipements sont actuellement soumis à différents traitements (chimiques ou thermochimiques), complémentaires à la désinfection et mis en oeuvre de façon alternée, afin d'obtenir une hygiène totale des circuits. Cette alternance est source d'erreurs, de contrainte pour les matériaux et nécessite une organisation adaptée.

Les inventeurs ont donc cherché à mettre au point un procédé chimique intégrant les différentes fonctions de l'hygiène totale, sans effets secondaires nuisibles et qui soit simple à mettre en oeuvre.

C'est pourquoi, l'invention a pour objet une composition comprenant pour 100 % de sa masse, essentiellement :
de 0,50% massique à 1,50% massique d'acide peracétique,
de 3,00% massique à 15,00% massique de peroxyde d'hydrogène,
de 3,00% massique à 15,00% massique d'acide acétique
de 0,10% massique à 1,00% massique d'acide nitrique concentré
de 0,001% massique à 0,20% massique d'agent tensioactif non ionique
de 0,01% massique à 0,10% massique d'oxyde d'amine,
de 0,01% massique à 0,20% massique d'agent stabilisant, et de l'eau pour compléter à 100% massique.

Comme agent tensioactif non ionique approprié à une mise en oeuvre dans la composition telle que définie précédemment, il y a notamment ceux formule (1) :

R₅-O-[CH(R₈)-CH(R₆)-O]ₙ-R₇ (I)

dans laquelle R₅ représente un radical aliphatique linéaire ou ramifié, saturé ou insaturé comportant de 5 à 31 atomes de carbone et de préférence de 10 à 16 atomes de carbone, R₆ représente un atome d'hydrogène, un radical méthyle ou un radical éthyle, R₈ représente un atome d'hydrogène, un radical méthyle ou un radical éthyle, étant entendu qu'au moins un des radicaux R₆ ou R₈ représente un atome d'hydrogène, R₇ représente un atome d'hydrogène, au un radical alkyle linéaire ou ramifié comportant de 1 to 4 atomes de carbone ou un radical benzyle et n représente un nombre compris entre 1 et 50, de préférence inférieur à 20. L'agent tensioactif non ionique est notamment choisi, parmi les produits commerciaux suivants : le GENAPOL^{™} EP 0244, le GENAPOL^{™} EP 2564, le GENAPOL^{™} EP 2584, le TRITON^{™} DF12, le TRITON^{™} DF16, le TRITON^{™} CF10 ou le SIMULSOL^{™} NW 900. Ces produits, disponibles dans le commerce, ont la composition chimique suivante :

| **Nom commercial** | **Composition chimique** |
|---|---|
| GENAPOL™ EP 0244 | Mélange d'alcools polyalcoxylés en C₁₀C₁₂, (4 OE, 4OP) |
| GENAPOL™ EP 2564 | Mélange d'alcools polyalcoxylés en C₁₂C₁₅ (6 OE, 4 OP) |
| GENAPOL™ EP 2584 | Mélange d'alcools polyalcoxylés en C₁₂C₁₅ (8 OE, 5OP) |
| TRITON™ DF12 | Ethers benzyliques d'alcools polyalcoxylés en C₈, C₁₀ (2OE, 5OP) |
| TRITON™ DF16 | Mélange d'alcools polyalcoxylés en C₈C₁₀ (6OE, 3OP) |
| TRITON™ DF10 | Ether benzylique d'alcools polyéthoxylés en C₈ (16OE) |
| SIMULSOL™ NW 900 | Mélange d'alcools polyalcoxylés (x OE, y OP) |

Comme oxyde d'amine appropriée à une mise en oeuvre dans la composition telle que définie précédemment, il y a notamment ceux formule (II):

(R₁)(R₂)(R₃)N → O (II)

dans laquelle R₁ représente un radical aliphatique linéaire or ramifié, comportant de 8 à 18 atomes de carbone et R₂ et R₃ représentent chacun un radical méthyle. Dans la formule (II) telle que R₁ est plus particulièrement choisi parmi les radicaux, octyle, décyle, dodécyle, tétradécyle or hexadecyle. Comme composés de formule (II) appropriés à la présente invention, il y a par exemple l'oxyde de cocodiméthylamine, comme celui commercialisé sous le nom AROMOX^{™} MCD-W, l'oxyde de myristamine ou l'oxyde de dihydroxyéthyl cocoamine.

Comme agent stabilisant approprié, pour de telles compositions, il y a par exemple les agents séquestrants et/ou les agents capteurs de radicaux libres comme ceux de la famille des butylhydroxytoluènes. On peut citer aussi les acides phosphoniques comme par exemple l'acide 1-hydroxyéthylidène-1,1-diphosphonique (HEDP).

L'eau mise en oeuvre dans la composition telle que définie précédemment est de préférence une eau conforme à la Pharmacopée européenne qui a subit un traitement qui comporte une succession d'étapes, comme la filtration, le passage sur résines échangeuses d'ions et pour terminer l'osmose inverse.

Selon un aspect particulier de la présente invention, la composition telle que définie précédemment comprend, pour 100 % de sa masse, essentiellement :
de 0,50% massique à 1,00 % massique d'acide peracétique,
de 4,00% massique à 10,00% massique de peroxyde d'hydrogène,
de 4,00% massique à 15,00% massique d'acide acétique
de 0,10% massique à 1,00% massique d'acide nitrique concentré
de 0,03% massique à 0,10% massique d'agent tensioactif non ionique
de 0,01% massique à 0,10% massique d'oxyde d'amine,
de 0,03% massique à 0,10% massique d'agent stabilisant, et de l'eau pour compléter à 100% massique.

Selon un autre aspect particulier de la présente invention, la composition telle que définie précédemment comprend, pour 100 % de sa masse, essentiellement :
de 0,70% massique à 1,00% massique d'acide peracétique,
de 4,00% massique à 7,00% massique de peroxyde d'hydrogène,
de 7,00% massique à 12,00% massique d'acide acétique,
de 0,20% massique à 0,70% massique d'acide nitrique concentré,
de 0,03% massique à 0,075% massique d'agent tensioactif non ionique
de 0,01% massique à 0,05% massique d'oxyde d'amine,
de 0,03% massique à 0,075% massique d'agent stabilisant, et de l'eau pour compléter à 100% massique.

L'invention aussi pour objet, un procédé de préparation de la composition telle que définie précédemment, caractérisé en ce qu'il comprend une étape de mélange pour 100% massique :
de 3% à 20% massique d'une solution aqueuse (A) comprenant de 5% massique à 30% massique d'acide peracétique, de 10% massique à 50% massique de peroxyde d'hydrogène, de 5% massique à 60% massique d'acide acétique, éventuellement jusqu'à 1 % d'agent stabilisant, et de l'eau pour compléter à 100% massique, avec
de 80% à 97% massique d'une solution aqueuse (B) préparée par mélange pour 100% massique :
   de 8% massique à 10% massique d'acide acétique,
   de 0,10% massique à 1% massique d'acide nitrique,
   de 3% massique à 10% massique de peroxyde d'hydrogène,
   de 0,03% massique à 0,1% massique d'agent tensioactif non ionique,
   de 0,01% massique à 0,1% massique d'oxyde d' amine,
   de 0,005% massique à 0,1% massique d'agent stabilisant et
   de l'eau pour compléter à 100% massique.

La solution (A) mise en oeuvre dans le procédé tel que défini ci-dessus est préparée avec de réactifs commerciaux tels que par exemple :
Le peroxyde d'hydrogène en solution aqueuse à 60% massique;
L'acide acétique glacial à 99% massique,
L'acide nitrique concentré à 58 % massique ;
et l'agent stabilisant de la famille du HEDP

On peut aussi utiliser une solution (A) à l'équilibre, telle que les solutions 15/23 (15 % massique d'acide peracétique ; 23 % de peroxyde d'hydrogène) ou 5/23 (5% massique d'acide peracétique ; 23% de peroxyde d'hydrogène).

On peut encore préparer la solution (A) mise en oeuvre dans le procédé tel que défini ci-dessus, par le procédé décrit dans la demande de brevet européen publiée sous le numéro EP 0 024 219, à partir de produits commerciaux.

L'invention a encore pour objet un procédé de nettoyage, de décalcification et de désinfection des circuits hydrauliques du générateur de dialyse entre deux séances de dialyse, caractérisé en ce qu'il comprend :
une étape (a) de circulation au sein des circuits hydrauliques dudit générateur de dialyse, pendant un temps compris entre 10 minutes et 30 minutes, d'une solution aqueuse obtenue par dilution entre le 1/20 et le 1/40 dans l'eau osmosée par ledit générateur, d'une composition telle que définie précédemment, et à l'issue de l'étape (a) ;
une étape (b) de rinçage consistant en la circulation au sein des circuits hydrauliques dudit générateur de dialyse, pendant un temps compris entre 15 minutes et 60 minutes, d'eau osmosée.

Par eau osmosée, on indique que l'eau mise en en oeuvre a préalablement subit un traitement de purification comportant une succession d'étapes, comme la filtration, le passage sur résines échangeuses d'ions, le passage sur charbon actif, et dont la dernière étape une purification par osmose inverse pour atteindre les valeurs des paramètres physico-chimiques et microbiologiques, exigées par les Pharmacopées française et européenne.

Selon un aspect particulier du procédé tel que défini ci-dessus, le taux de dilution de la composition aqueuse mise en oeuvre est compris entre 1/25 et le 1/35.

L'invention a encore pour objet un procédé de nettoyage, de décalcification et de désinfection de la boucle de distribution de la chaîne de traitement d'eau pour hémodialyse, caractérisé en ce qu'il comprend :
une étape (a) de circulation au sein de ladite boucle de distribution, pendant un temps compris entre 30 minutes et 60 minutes, d'une solution aqueuse obtenue par dilution entre le 1/20 et le 1/40 dans l'eau d'une composition telle que définie à l'une des revendications 1 à 3, et à l'issue de l'étape (a),
une étape (b) de rinçage à l'issue de l'étape (a), consistant en la circulation au sein de ladite boucle de distribution, pendant un temps compris entre 30 minutes et 60 minutes, d'eau osmosée.

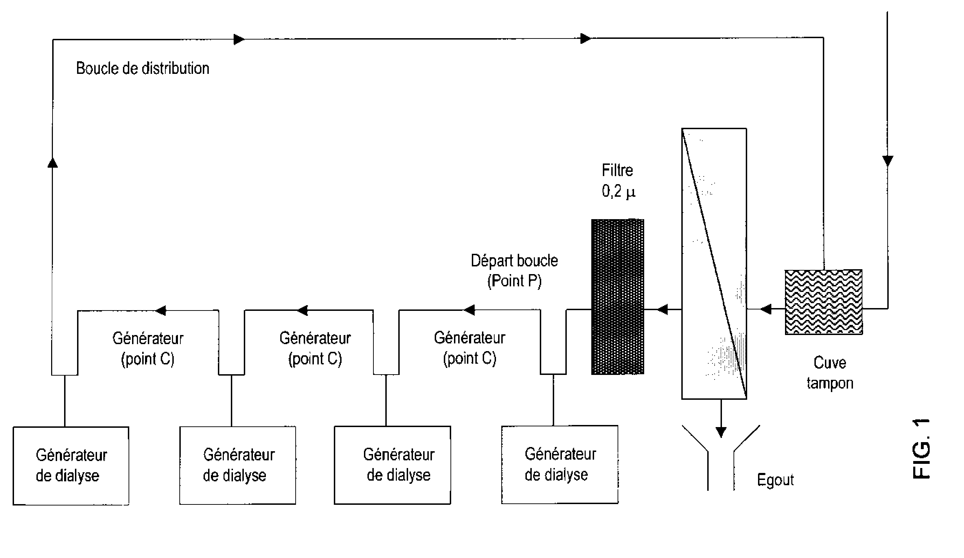

Une boucle de distribution d'eau pour hémodialyse est illustrée par la figure 1.

L'invention a enfin pour objet l'utilisation de la composition telle que définie précédemment, pour nettoyer, décalcifier et désinfecter des circuits hydrauliques du générateur de dialyse en une seule opération entre deux séances successives de dialyse, sans traitement complémentaire autre qu'un rinçage dudit équipement de dialyse avec de l'eau osmosée.

L'exposé expérimental suivant illustre l'invention sans toutefois la limiter.

### A) Préparation d'une compositon 1 selon l'invention :

La composition 1 comportant 0,99% massique d'acide peracétique, 6,90% massique de peroxyde d'hydrogène, 10,40% massique d'acide acétique, 0,58% massique d'acide nitrique, 0,05% massique de GENAPOL^{™} EP 2564 (tensioactif non ionique), 0,015% massique d'oxyde de cocodiméthylamine, 0,07% massique d'acide 1-hydroxyéthylidène-1,1-diphosphonique et de l'eau pour compléter à 100% massique, est préparée pour (100% massique) par mélange de 22% massique d'une solution aqueuse (A1) comportant pour 100% de sa masse :
entre 4,50% et 5,10% massique d'acide peracétique,
entre 12,00% et 13,00% massique d'acide acétique,
entre 25,00% et 27,00% massique de peroxyde d'hydrogène,
environ 0,50% massique d'une solution d'acide 1-hydroxyéthylidène-1,1-diphosphonique (à 60% massique),
avec 88% massique d'une solution (B1) préparée, pour 100% de sa masse, à partir de:
entre 8,00% et 9,00% massique d'acide acétique glacial à 99% massique,
entre 3,80% et 4,00% massique de peroxyde d'hydrogène à 60% massique
entre 8,50% et 9,00% massique d'acide nitrique concentré à 58% massique
entre 0,05% et 0,06% massique de GENAPOL^{™} EP 2564
entre 0,05% massique et 0,06% massique d'AROMOX^{™} MCD-W) (oxyde de cocodiméthylamine à 30% massique), et
environ 0,006% massique d'une solution d'acide 1-hydroxyéthylidène-1,1-diphosphonique à 60% massique).

### B) Analyse des propriétés désinfectantes de la composition 1:

On a effectué les essais recensés dans le tableau suivant, afin de valider les efficacités bactéricide, fongicide et virucide de la composition 1 en les comparant avec celle d'une solution aqueuse à l'équilibre comprenant 0,42% massique d'acide peracétique, 7% massique de peroxyde d'hydrogène et 3,5% à 4,5 % massique d'acide acétique, et qui est utilisée depuis plusieurs années dans les centres de dialyse, comme désinfectant et détartrant des générateurs de dialyse. Certains essais sont effectués à une dilution au 35^{ème} correspondant à celle mise en oeuvre dans le générateur AK 200 Ultra^{™} de la société Gambro, en suivant les prescriptions des normes européenne ou française, pertinentes en l'espèce. Les résultas sont consignés dans les tableaux suivants.

| BACTERICIDIE | | |
|---|---|---|
| Temps de contact (Tc) Température (T) Conditions opératoires (CO) | DIALOX^{™} | Composition selon l'invention |
| (Essai selon la Norme NF EN 1040) | | |
| Tc : 5 minutes ; T : 20°C ; | CMB : 0,4% | CMB : 0,2% |
| CO : Absence de substance interférente | (18 ppm APA) | (20 ppm APA) |

| (Essai selon la Norme NF EN 13727) | | |
|---|---|---|
| Tc : 5 minutes ; T : 20°C | CMB : 2,0% | CMB : 0,4% |
| CO : Condition de propreté | (90 ppm APA) | (40 ppm APA) |
| | | |
| Tc : 60 minutes ; T : 20°C | CMB : 2,0% | CMB : 0,4% |
| CO : Condition de propreté | (90 ppm APA) | (40 ppm APA) |
| Tc : 5 minutes ; T: 20°C | - | CMB : 1% |
| CO : Condition de saleté | | (100 ppm APA) |
| | | |
| Tc : 60 minutes ; T : 20°C ; | | CMB : 1% |
| CO : Condition de saleté | - | (100 ppm APA) |
| | | |
| Tc : 5 minutes ; T : 37 °C ; | | CMB : 0,2% |
| CO : Condition de propreté | - | (20ppm APA) |
| | | |
| Tc : 5 minutes ; T : 37 °C ; | - | CMB : 1% |
| CO : Condition de saleté | | (100 ppm APA) |

| FONGICIDIE | | |
|---|---|---|
| Temps de contact (Tc) | DIALOX^{™} | Composition selon l'invention |
| Température (T) | | |
| Conditions opératoires (CO) | | |

| Norme NF EN 1275 | | |
|---|---|---|
| Tc : 15 minutes ; T : 20°C ; | CMF : 5,0% | CMF : 2,85 % |
| CO : Absence de substance interférente | (225 ppm APA) | (285 ppm APA) |

| Norme NF EN 13624 | | |
|---|---|---|
| Tc : 10 minutes ; T : 20°C | | CMF : 4,0 % |
| CO : Condition de propreté | | (400 ppm APA) |
| | | |
| Tc : 15 minutes ; T : 20°C ; | CMF : 5,0% | CMF :3,0 % |
| CO : Condition de propreté | (225 ppm APA) | (300 ppm APA) |
| | | |
| Tc : 60 minutes ; T : 20°C ; | CMF : 5,0% | CMF : 2,0% |
| CO : Condition de propreté | (225 ppm APA) | (200 ppm APA) |
| | | |
| Tc : 10 minutes ; T : 20°C ; | | CMF : 6% |
| CO : Condition de saleté | - | (600 ppm APA) |
| | | |
| Tc : 15 minutes ; T : 20°C ; | - | CMF : 4% |
| CO : Condition de saleté | | (400 ppm APA) |
| | | |
| Tc : 60 minutes ; T : 20°C ; | - | CMF : 2,85% |
| CO : Condition de saleté | | (285 ppm APA) |
| | | |
| Tc : 10 minutes; T : 37°C ; | - | CMF : 2,85% |
| CO : Condition de propreté | | (285 ppm APA) |
| | | |
| Tc : 15 minutes; T : 37°C ; | - | CMF : 2,0% |
| CO : Condition de propreté | | (200 ppm APA) |
| | | |
| Tc : 10 minutes; T : 37°C ; | - | CMF : 4,0% |
| CO : Condition de saleté | | (400 ppm APA) |
| | | |
| Tc : 15 minutes; T : 37°C ; | - | CMF : 2,85% |
| CO : Condition de saleté | | (285 ppm APA) |

| | | |
|---|---|---|
| CMB : Concentration minimale bactéricide ; APA : acide peracétique ; Condition de propreté : 0,03% d'albumine ; Condition de saleté : 0,3% d'albumine + 0,3% d'érythrocytes de sang de mouton ; CMF : Concentration minimale fongicide ; La concentration de 2,85% correspond aux conditions d'emploi dans la machine (= dilution au 35^{ème}) | | |

| VIRUCIDIE | | |
|---|---|---|
| Temps de contact (Tc) | DIALOX^{™} | Composition selon |
| Température (T) | | l'invention |
| Conditions opératoires (CO) | | |
| Norme NFT 72-180 | | |
| Tc : 15 minutes ; T : 37°C ; | CMV : 4,0% | CMV : 1,5 % |
| (Adénovirus type 5 cultivé sur cellule HeLa) | | |

Les résultats mettent en évidence que dans les conditions d'utilisation en générateurs (dilution au 35^{ème}), la composition 1 selon l'invention est bactéricide selon la norme NF EN 13727 et fongicide selon NF EN 13624 en 60 minutes à 20°C dans des conditions de propreté, 60 minutes à 20°C dans les conditions de saleté, 10 minutes à 37°C dans les conditions de propreté et 15 minutes à 37°C dans les conditions de saleté, tout en conservant le classement produit irritant (acide peracétique < 1% et peroxyde d'hydrogène < 7%). Ils mettent aussi en évidence que la composition 1 selon l'invention est virucide selon la norme NFT 72-180

### C) Essais de la composition 1 en générateur de dialyse :

### 1) Test de rinçabilité

Une première série d'essais a été effectuée sur un générateur de dialyse **AK 200 Ultra™** de la société **Gambro** dont les paramètres liés à la désinfection sont les suivants : volume de désinfectant aspiré : 120 ml ; temps de désinfection : 10 minutes (température comprise entre 37°C et la température ambiante) ; durée totale du cycle (désinfection + rinçage) : 41 mn.

On vérifie visuellement l'absence de mousse pendant et après la phase de désinfection. Le cycle de désinfection se déroule normalement et aucune trace de mousse n'est visible à la fin du rinçage. On vérifie à l'issue de l'étape de rinçage l'absence de toute trace de désinfectant dans le dialysât avant la séance de dialyse. Le dialysât est préparé par le générateur après une période de test succédant à la phase de rinçage du désinfectant. Les résultats sont les suivants sur la base des divers paramètres pris comme indicateurs compte tenu de la nature des constituants permettant cette hygiène totale des surfaces :

| | H₂O₂ à la fin du lavage (évacuation) | H₂O₂ à la fin de la période de test | H₂O₂ dans le dialysât | Tension superficielle en mN/m (dialysât) | Conductivité en µS / cm à la fin de la période de test |
|---|---|---|---|---|---|
| DIALOX | 2 ppm | 0 | 0 | 69,5 mN/m | 4,2 µS / cm |
| Composition 1 (essai 1) | 5 ppm | 0 | 0 | 68,5 mN/m | 4,2 µS / cm |
| Composition 1 (essai 2) | 2 ppm | 0 | 0 | 70,1 mN/m | 4,2 µS/cm |

L'absence de toute trace de désinfectant est démontrée. La conductivité initiale (solution désinfectante) étant d'environ 1200 µS/cm (pour une dilution au 35^{ème}), ce paramètre peut être utilisé comme indicateur avant le démarrage de la désinfection et comme indicateur de fin de rinçage. Ces résultats mettent en évidence la bonne rinçabilité de la composition 1 selon l'invention.

### 2) Validation de la rinçabilité de la composition 1 en conditions d'utilisation sur une période de 5 jours

Une deuxième série d'essais est réalisée sur une période de 5 jours pour apprécier la bonne rinçabilité de la composition 1 selon l'invention au sein des circuits hydrauliques du générateur de dialyse **AK 200 Ultra™** de la société **Gambro** dans les mêmes conditions expérimentales que celles exposées au paragraphe précédent.

La composition 1 est mise en oeuvre durant cette période deux fois par jour avec le même générateur de dialyse, ledit générateur étant maintenu en stagnation entre deux essais, avec de l'eau osmosée au sein de ses circuits hydrauliques.

Des prélèvements d'eau de rinçage sont analysés quotidiennement. Ils permettent d'attester de la rinçabilité constante et permanente de la composition 1 selon l'invention.

La même série de test a été réalisée sur un générateur FRESENIUS 4000 H^{™} de la société Frésénius opérant avec une dilution au 25^{ème}, pendant une période de 5 jours.. Ils ont conduit à des résultats eux aussi conforme aux critères de rinçabilité exigés.

### 3)- Validation du pouvoir nettoyant et desinfectant de la composition 1 selon l'invention sur une période de 9 mois

La composition 1 est mise en oeuvre durant cette période entre chaque dialyse avec sur une période de neuf mois dans une batteries de 16 générateurs de dialyse **AK 200 Ultra^{™}** de la société **Gambro,** fonctionnant en hémodiafiltration.. La composition 1 diluée au 35^{ème} dans la machine est laissée en stagnation dans les circuits du générateur chaque nuit entre les jours de séances de dialyse. Des prélèvements d'eau de rinçage sont analysés quotidiennement. On prélève des échantillons de dialysât tous les deux jours en différents endroits des circuits pour une analyse bactériologique (gélose R2A T 20/232°C) et une recherche d'endotoxines (Gel point final avec dilutions successives à la moitié ; Sensibilité du réactif : 0,015UI/ml). Ils conduisent aux résultats consignés dans le tableau suivant :

| | Départ de la boucle | Retour de la boucle | Dialysat hémodiafiltration | Réinjection hémodiafiltration |
|---|---|---|---|---|
| Bactériologie à 48 heures | R.A.S. | R.A.S. | R.A.S. | R.A.S. |
| | Absence de germes pathogènes | | | |
| Bactériologie à 7 jours | R.A.S. | R.A.S. | R.A.S. | R.A.S. |
| | Absence de germes pathogènes | | | |
| Endotoxines | < 0,015 UI/ml | < 0,015 UI/ml | < 0,015 UI/ml | R.A.S. |

| | | | | |
|---|---|---|---|---|
| R.A.S. : Rien à signaler | | | | |

Ils mettent en évidence l'absence de bactérie ou d'endotoxines. Des observations visuelles confirment l'absence de tartre et/ ou de résidus solides dans le circuit.

La mise en oeuvre d'une composition 1 selon l'invention pour traiter les appareils de dialyse conduit donc aux avantages suivants :
- la réduction du temps d'immobilisation de l'équipement de dialyse entre deux séances ;
- l'élimination de toute souillure de nature organique par application itérative c'est à dire entre chaque patient ;
- l'élimination de toute souillure de nature minérale par application itérative c'est à dire entre chaque patient ;
- une sécurité supplémentaire pour une hygiène totale grâce à la conductivité suffisante de la solution aqueuse diluée pour être exploitée comme traceur (en désinfection et après rinçage) par l'équipement de dialyse ;
- la conformité aux nouvelles normes d'évaluation de l'activité désinfectante pour les désinfectants des circuits de générateurs de dialyse ;
- le classement du produit comme simplement irritant de par la synergie d'action de ses constituants ;
- l'innocuité vis à vis du matériel ;
- l'absence de mousse et de tout résidu après la phase de rinçage ;

## Revendications

1. Composition comprenant pour 100 % de sa masse, essentiellement :
de 0,50% massique à 1,50 % massique d'acide peracétique,
de 3,00% massique à 15,00% massique de peroxyde d'hydrogène,
de 3,00% massique à 15,00% massique d'acide acétique
de 0,10% massique à 1,00% massique d'acide nitrique concentré
de 0,001% massique à 0,20% massique d'agent tensioactif non ionique
de 0,01% massique à 0,10% massique d'oxyde d'amine,
de 0,01% massique à 0,20% massique d'agent stabilisant, et
de l'eau pour compléter à 100% massique.

2. Composition telle que définie à la revendication 1, comprenant pour 100 % de sa masse essentiellement :
de 0,50% massique à 1,00% massique d'acide peracétique,
de 4,00% massique à 10,00% massique de peroxyde d'hydrogène,
de 4,00% massique à 15,00% massique d'acide acétique,
de 0,10% massique à 1,00% massique d'acide nitrique concentré,
de 0,03% massique à 0,10% massique d'agent tensioactifnon ionique,
de 0,01% massique à 0,10% massique d'oxyde d'amine,
de 0,03% massique à 0,10% massique d'agent stabilisant, et
de l'eau pour compléter à 100% massique.

3. Composition telle définie à la revendication 2, comprenant pour 100% de sa masse essentiellement :
de 0,70% massique à 1,00% massique d'acide peracétique,
de 4,00% massique à 7,00% massique de peroxyde d'hydrogène,
de 7,00% massique à 12,00% massique d'acide acétique,
de 0,20% massique à 0,70% massique d'acide nitrique concentré,
de 0,03% massique à 0,075% massique d'agent tensioactif non ionique,
de 0,01% massique à 0,05% massique d'oxyde d'amine,
de 0,03% massique à 0,075% massique d'agent stabilisant, et
de l'eau pour compléter à 100% massique.

4. Procédé de préparation de la composition telle que définie à l'une des revendications 1 ou 2, **caractérisé en ce qu'**il comprend une étape de mélange pour 100% massique :
de 3% à 20% massique d'une solution aqueuse (A) comprenant de 5% massique à 30% massique d'acide peracétique, de 10% massique à 50% massique de peroxyde d'hydrogène, de 5% massique à 60% massique d'acide acétique, éventuellement jusqu'à 1% d'agent stabilisant, et de l'eau pour compléter à 100% massique, avec
de 80% à 97% en volume d'une solution aqueuse (B) préparée par mélange pour 100% massique :
de 8% massique à 10% massique d'acide acétique,
de 0,10% massique à 1% massique d'acide nitrique,
de 3% massique à 10% massique de peroxyde d'hydrogène,
de 0,03% massique à 0,1% massique d'agent tensioactif non ionique,
de 0,01% massique à 0,1% massique d'oxyde d' amine,
de 0,005% massique à 0,1% massique d'agent stabilisant et
de l'eau pour compléter à 100% massique.

5. Procédé de nettoyage, de décalcification et de désinfection des circuits hydrauliques du générateur de dialyse entre deux séances de dialyse, **caractérisé en ce qu'**il comprend :
une étape (a) de circulation au sein des circuits hydrauliques dudit générateur de dialyse, pendant un temps compris entre 10 minutes et 30 minutes, d'une solution aqueuse obtenue par dilution entre le 1/20 et le 1/40 dans l'eau osmosée par ledit générateur, d'une composition telle que définie à l'une des revendications 1 ou 2, et à l'issue de l'étape (a) ;
une étape (b) de rinçage consistant en la circulation au sein des circuits hydrauliques dudit générateur de dialyse, pendant un temps compris entre 15 minutes et 60 minutes, d'eau osmosée.

6. Procédé tel que défini à la revendication 5, dans lequel le taux de dilution de la composition aqueuse mise en oeuvre est compris entre 1/25 et le 1/35.

7. Procédé de nettoyage, de décalcification et de désinfection de la boucle de distribution de la chaîne de traitement d'eau pour hémodialyse, **caractérisé en ce qu'**il comprend :
une étape (a) de circulation au sein au sein de ladite boucle de distribution, pendant un temps compris entre 30 minutes et 60 minutes, d'une solution aqueuse obtenue par dilution entre le 1/20 et le 1/40 dans l'eau d'une composition telle que définie à l'une des revendications 1 à 3, et à l'issue de l'étape (a) ;
une étape (b) de rinçage à l'issue de l'étape (a), consistant en la circulation au sein au sein de ladite boucle de distribution, pendant un temps compris entre 30 minutes et 60 minutes, d'eau osmosée.

8. Utilisation de la composition telle que définie à l'une des revendications 1 à 3, pour nettoyer, de décalcifier et désinfecter des équipements de dialyse entre deux séances successives de dialyse, sans traitement complémentaire autre qu'un rinçage dudit équipement de dialyse avec de l'eau osmosée.
